# EUROPEAN PATENT APPLICATION

(11) **EP 2 436 350 A2**
(43) Date of publication of application: **04.04.2012**
(21) Application number: 11182692.1
(22) Date of filing: 26.09.2011
(51) Int. Cl.: A61F 13/08

(54) **Compression Garment Having Grip**

(30) Priority: 29.09.2010 US 893686
(71) Applicant: Tyco Healthcare Group LP, Mansfield, MA 02048 (US)
(72) Inventor: Deshpande, Manish, Canton, Massachussets 02021 (US); Bruehwiler, Michel, Newton, Massachussets 02461 (US); Rosen, Melissa, Salem Massachussets, 01970 (US)
(74) Representative: Gray, James

(57) **Abstract**

A compression garment for providing compression therapy to a body part. The garment includes a flexible wrap and a bladder held by the wrap for compressing the body part. The garment also includes a fastener for holding the wrap on the body part in a self-retaining configuration. A grip is provided to facilitate tightening of the wrap on the body part. In one embodiment, the grip comprises an opening in the wrap sized and shaped for receiving a finger of a user.

## Description

### FIELD OF THE INVENTION

The present invention generally relates to a compression garment, and more particularly to a compression garment having a grip for facilitating application of the garment to a body part.

### BACKGROUND OF THE INVENTION

A major concern for generally immobile patients and like persons are medical conditions that form blood clots, such as, deep vein thrombosis (DVT) and peripheral edema. Such patients and persons include those undergoing surgery, anesthesia, extended periods of bed rest, etc. These blood clotting conditions generally occur in the deep veins of the lower extremities and/or pelvis. These veins, such as the iliac, femoral, popliteal, and tibial return deoxygenated blood to the heart. For example, when blood circulation in these veins is retarded due to illness, injury, or inactivity, there is a tendency for blood to accumulate or pool. A static pool of blood may lead to the formation of a blood clot, which can interfere with cardiovascular circulation. Most seriously, a fragment of the blood clot can break loose and migrate. A pulmonary embolus can form from the fragment potentially blocking a main pulmonary artery, which may be life threatening. The current invention can also be applied to the treatment of other conditions, such as lymphedema.

Conventional vascular compression systems include a compression garment fluidly connected to a controller for cyclically inflating the compression garment. The cyclical inflation of the compression garment enhances blood circulation and decreases the likelihood of DVT. A system of conduits connects the compression garment to the controller. Newer vascular compression garments have portable controllers that are much smaller and even mountable on the compression garment so that the patient may move about freely without having to first remove the compression garment or disconnect the compression garment from a controller. These new compression garments may be worn when a patient is stationary or ambulatory and enhance patient compliance because of convenience of use.

Active compression garments for applying intermittent compression therapy to a body part (e.g., a limb such as a leg) have many applications, including DVT prophylaxis, edema prevention, and aiding in wound healing. The performance of such compression garments is sensitive to the initial fit or tightness of the garment, the ability of the garment to retain its fit and tightness, and the ability of the inflatable bladders to retain their original position around the body part. This can be very difficult when the compression garments are used during and after ambulation, such as walking, sitting, standing, and rolling over. The garments tend to slide down the body part causing misalignment of inflatable bladders with corresponding body parts, which may result in ineffective compression therapy and/or discomfort. The present invention is directed to facilitating application of the garment to a body part to obtain a desired fit to improve applied compression therapy.

### SUMMARY OF THE INVENTION

One aspect of the present invention is directed to a compression garment adapted for placement on a body part for providing compression therapy to the body part. The compression garment includes a flexible wrap having a proximal end sized for wrapping around a proximal portion of the body part, a distal end opposite the proximal end for wrapping around a distal portion of the body part, and lateral side edges extending between the proximal end and the distal end. A bladder is held by the wrap for compressing the body part. The bladder has an inflatable chamber and a port in fluid communication with the chamber for selectively delivering fluid from a fluid source to the chamber to inflate the chamber and compress the body part. At least one of the side edges defines a flap having a free end for holding the wrap on the body part in a self-retaining configuration in which the proximal end is wrapped around the proximal portion of the body part, the distal end is wrapped around the distal portion of the body part, and the lateral side edges extend generally longitudinally along the body part between the proximal end and the distal end. A fastener on the wrap secures the free end of the flap on the compression garment for holding the wrap on the body part in said self-retaining configuration. A grip is formed on the side edge opposite the flap for gripping the respective side edge when wrapping the wrap around the body part and fastening the fastener to hold the free end of the flap on the compression garment for holding the wrap on the body part. The grip is positioned on the wrap so that it is longitudinally offset with respect to the flap, permitting the flap to be fastened over the side edge having the grip without lapping the flap over the grip.

Another aspect of the invention is directed to a compression garment adapted for placement on a body part for providing compression therapy to the body part. The compression garment includes a flexible wrap having a proximal end sized for wrapping around a proximal portion of the body part, a distal end opposite the proximal end for wrapping around a distal portion of the body part, and lateral side edges extending between the proximal end and the distal end. A bladder is held by the wrap for compressing the body part, the bladder having an inflatable chamber and a port in fluid communication with the chamber for selectively delivering fluid from a fluid source to the chamber to inflate the chamber and compress the body part. At least one side edge defines a flap having a free end for holding the wrap on the body part in a self-retaining configuration in which the proximal end is wrapped around the proximal portion of the body part, the distal end is wrapped around the distal portion of the body part, and the lateral side edges extend generally longitudinally along the body part between the proximal end and the distal end. A fastener on the wrap secures the free end of the flap on the compression garment for holding the wrap on the body part in said self-retaining configuration. A hole extends entirely through the wrap in a margin of the wrap adjacent the side edge of the wrap opposite the flap. The hole is sized and shaped for receiving a finger of a user for gripping the respective side edge when wrapping the wrap around the body part and fastening the flap to hold the wrap on the body part.

Another aspect of the invention is directed to a method of placing a compression garment on a body part for providing compression therapy to the body part. The compression garment includes a bladder having an inflatable chamber and a port for providing fluid communication with the inflatable chamber. The method includes wrapping the compression garment around the body part such that a first side margin of the compression garment overlaps a second side margin of the compression garment and inserting a first finger into a first opening in the second side margin to grip the second side margin. The method further includes pulling at least one of the first side margin and the second side margin while gripping the first side margin to tighten the compression garment around the body part and securing the tightened compression garment around the body part.

Other objects and features will be in part apparent and in part pointed out hereinafter.

### BRIEF DESCRIPTION OF THE DRAWINGS

FIG. 1 is a schematical front elevation of a compression garment of the present invention shown in an open, unwrapped configuration;
FIG. 2 is a schematical rear elevation of the compression garment;
FIG. 3 is a schematical side perspective of the compression garment in a wrapped configuration; and
FIG. 4 is a schematical fragmentary elevation of flaps of the compression garment in an overlapped configuration.

Corresponding reference characters indicate corresponding parts throughout the drawings.

### DETAILED DESCRIPTION

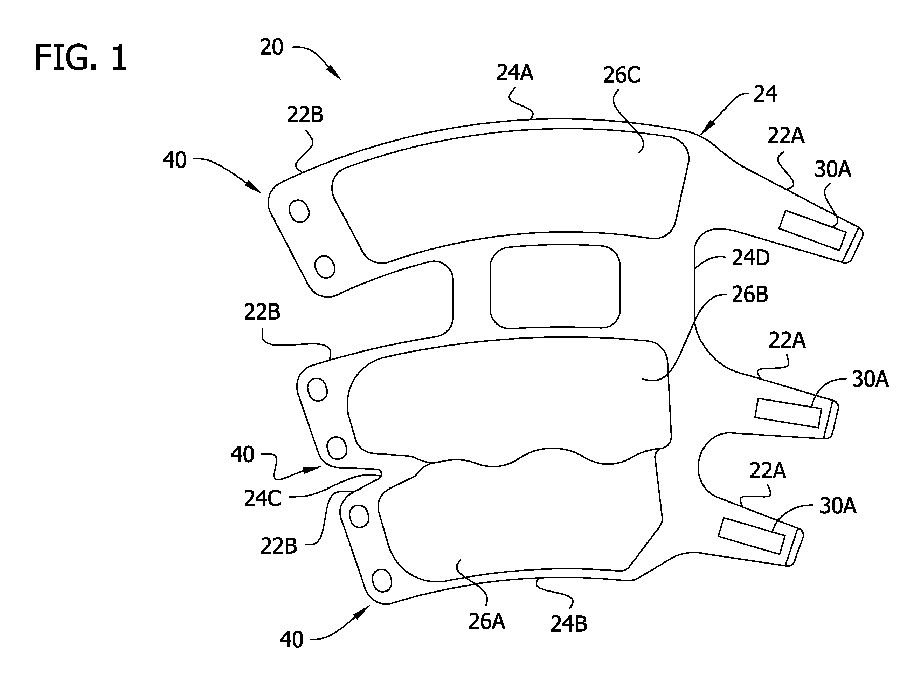

Referring to the drawings and in particular to FIGS. 1-3, a compression garment for applying compression therapy to a body part of a wearer is generally indicated by the reference number 20. The compression garment 20 is adapted for placement on the body part (e.g., a leg or arm). The illustrated compression garment 20 is adapted for placement on a leg. The compression garment 20 may be used to provide compression therapy such as constant or intermittent compression to the body part.

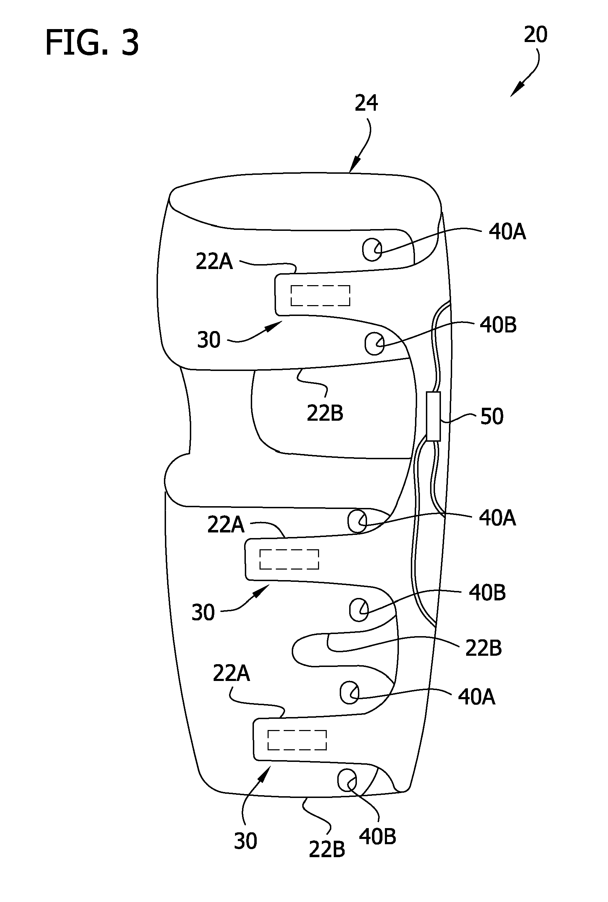

The compression garment 20 includes a flexible wrap, generally designated by 24, and three bladders 26A-26C. The wrap 24 is configured for holding the bladders 26A-26C for compressing the leg while the wrap is in a self-retaining configuration (FIG. 3). The wrap 24 has a proximal end 24A sized for wrapping around a proximal portion of the leg, a distal end 24B opposite the proximal end for wrapping around a distal portion of the leg, and lateral side edges 24C, 24D extending between the proximal end and the distal end.

The illustrated compression garment 20 has a "thigh length" size, i.e., the compression garment extends generally from the ankle to the thigh. The bladders 26A-26C include a conventional inflatable chamber and a port in fluid communication with the chamber for selectively delivering fluid from a fluid source to the chamber to inflate the chamber and compress the leg. The bladders 26A-26C are positioned on the wrap 24 to generally overlie the rear side of the leg, and more particularly, the ankle, calf, and thigh, respectively. The three bladders 26A-26C are arranged to lie in sequence along the leg. Other sizes and shapes of garments 20 (e.g., "knee length," extending generally from the ankle to below the knee) or having different configurations of bladders 26A-26C (e.g., one, two, or more bladders) are within the scope of the present invention.

The compression garment 20 may be placed in the self-retaining configuration on the leg by positioning the bladders 26A-26C over desired compression zones on the leg and wrapping the compression garment around the leg such that the side edges 24C, 24D or side margins of the compression garment overlap each other. In the self-retaining configuration, the proximal end 24A is wrapped around the proximal portion of the leg, the distal end 24B is wrapped around the distal portion of the leg, and the lateral side edges 24C, 24D extend generally longitudinally along the leg between the proximal end and the distal end. A generally tight or snug fit is desirable so that when the bladders 26A-26C are pressurized the leg is compressed. In the illustrated embodiment, the side edges 24C, 24D define three sets of corresponding flaps 22A, 22B spaced from each other. The flaps 22A, 22B facilitate tightening and securing of the compression garment around the leg. The compression garment 20 is tightened around the leg by pulling and overlapping the opposite side margins the compression garment. In the illustrated embodiment, each flap 22B is overlapped by a respective flap 22A. Thus, flaps 22A and 22B may be referred to as outer and inner flaps, respectively. Other configurations of flaps are envisioned as being within the scope of the present invention. For example, the garment may include fewer or more flaps, and the flaps 22B may be omitted so that the outer flaps 22A overlap the side margin corresponding to the side edge 24D.

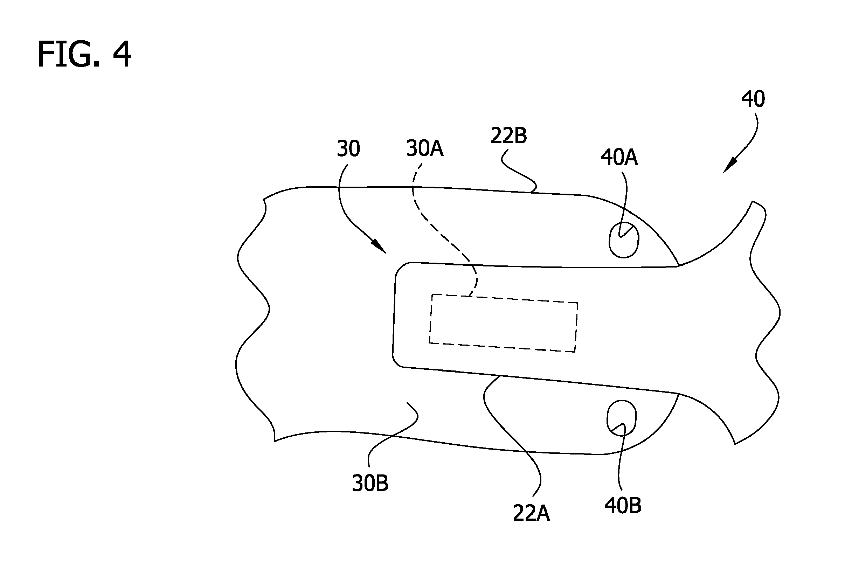

Referring to FIGS. 3 and 4, fasteners 30 on the wrap 24 are used to secure the free ends of the flaps 22A on the compression garment 20. In the illustrated embodiment, the fasteners 30 comprise hook fabric 30A on the free end of the flaps 22A. The fasteners 30 also comprise loop fabric 30B on an outside surface of the flaps 22B. Thus, the fasteners 30 are two-part fasteners. Other types of fasteners may be used without departing from the scope of the present invention.

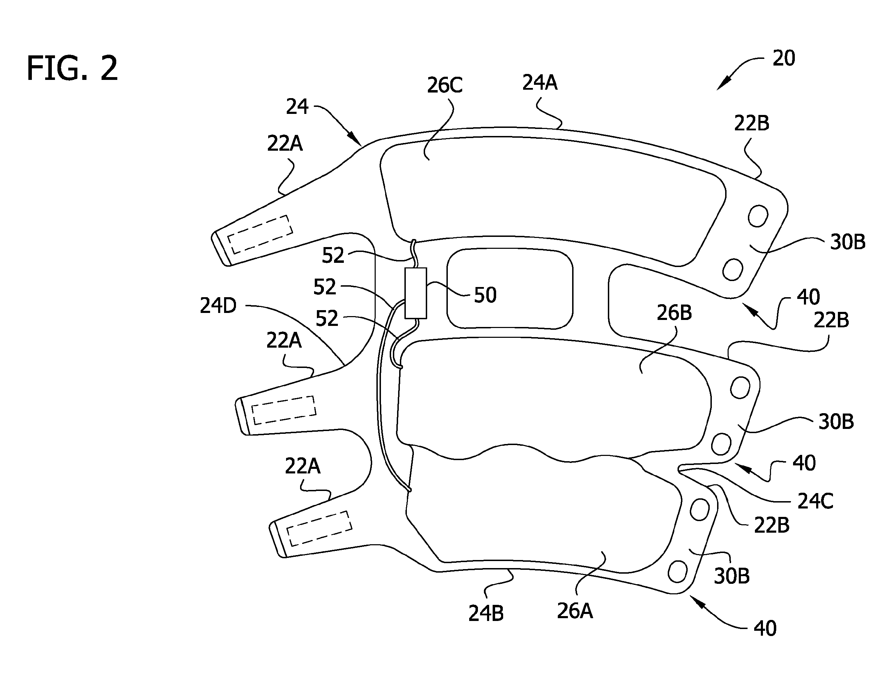

As shown in FIGS. 1 and 2, the compression garment 20 includes grips, generally designated by 40, formed on the side edge 24C opposite the flaps 22A for gripping the respective side edge 24C when wrapping the wrap 24 around the leg and fastening the flaps 22A to hold the wrap on the leg. Referring to FIGS. 3 and 4, each grip 40 comprises two openings and more specifically a pair of holes 40A, 40B extending entirely through the wrap sized and shaped for receiving a finger of a user to hold the wrap in place on the leg. The holes 40A, 40B are positioned on the flaps 22B or the side margin of the wrap adjacent the side edge of the wrap 24C opposite the flaps 22A. The holes 40A, 40B are longitudinally offset from the flaps 22A, permitting the flaps 22A to be fastened over the side edge 24C having the grips without lapping the flaps 22A over respective holes 40A, 40B. The holes 40A, 40B are spaced from each other by a distance sufficient to receive the flaps 22A between the holes. Accordingly, the grips 40 are positioned to facilitate tightening of the wrap 24 and to facilitate holding the flaps 22B in place as the flaps 22A are fastened. Thus, the grips 40 are accessible when the flaps 22A overlap and are fastened to the flaps 22B. Other types of grips are within the scope of the present invention. For example, the grip may comprise more or fewer openings, and openings that do not pass entirely through the garment may be used. Further, it is envisioned that the grips may comprise features having shapes different from those illustrated in the drawings.

The compression garment 20 may be constructed in various ways known in the art. In one construction, the garment 20 is formed from opposing inner and outer sheets of a generally flexible, fluid impervious material (e.g., PVC) that are welded together along bladder lines to form the bladders 26A-26C. Alternatively, the bladders 26A-26C may be formed separately and mounted on the compression garment 20.

As shown in FIG. 2, the compression garment 20 may include a pressurizer 50 operatively connectable to the ports of the bladders 26A-26C such as by conduits 52 for inflating the bladders. The illustrated pressurizer 50 is mounted on the compression garment 20. A configuration in which a pressurizer is removably mounted on a compression garment and operatively connected to bladders on the compression garment is disclosed in more detail in U.S. Patent Applications 12/241,670 and 12/241,936, both of which are assigned to Tyco Healthcare Group LP and incorporated by reference in their entireties. Other embodiments, such as where the pressurizer 50 is not configured for mounting on the compression garment 20 are envisioned as being within the scope of the present invention.

The pressurizer 50 may be programmed to execute various compression regimens, which may include inflation and vent phases. In some regimens, the pressurizer may pressurize one or more of the bladders 26A-26C to a constant pressure for an extended time period. The pressurizer 50 may also intermittently or cyclically pressurize the bladders 26A-26C. Other types of compression regimens are within the scope of the present invention.

In a cycle of use, the compression garment 20 is placed on a body part such as the leg, with the bladders 26A-26C covering or overlying a desired target compression zone. The wrap 24 is wrapped around the body part such that the side margins of the wrap adjacent the side edges 24C, 24D overlap each other. A person uses the grips 40 by inserting fingers such as a thumb and a forefinger into respective holes 40A, 40B to grip the flaps 22B. While gripping a flap 22B using the finger holes 40A, 40B, at least one of the side margins and in particular one of the flaps 22A, 22B is moved with respect to the other of the side margins to tighten the compression garment around the leg. The respective flap 22A is pulled between the fingers inserted in the openings 40A, 40B and fastened to the outside surface of the flap 22B using the fastener 30. These steps are repeated for each set of corresponding flaps 22A, 22B. The grips 40 thus facilitate tightening of the compression garment 20 on the leg and holding of the flaps 22B in place while the flaps 22A are fastened to the outer surface of the flaps 22B. The tightened compression garment 20 results in an improved fit for improved compression therapy.

When introducing elements of the present invention or the preferred embodiments(s) thereof, the articles "a", "an", "the", and "said" are intended to mean that there are one or more of the elements. The terms "comprising", "including", and "having" are intended to be inclusive and mean that there may be additional elements other than the listed elements.

In view of the above, it will be seen that the several objects of the invention are achieved and other advantageous results attained.

## Claims

1. A compression garment adapted for placement on a body part for providing compression therapy to the body part, the compression garment comprising:
a flexible wrap having a proximal end sized for wrapping around a proximal portion of the body part, a distal end opposite the proximal end for wrapping around a distal portion of the body part, and lateral side edges extending between the proximal end and the distal end;
a bladder held by the wrap for compressing the body part, the bladder having an inflatable chamber and a port in fluid communication with the chamber for selectively delivering fluid from a fluid source to the chamber to inflate the chamber and compress the body part;
at least one of the side edges defining a flap having a free end for holding the wrap on the body part in a self-retaining configuration in which the proximal end is wrapped around the proximal portion of the body part, the distal end is wrapped around the distal portion of the body part, and the lateral side edges extend generally longitudinally along the body part between the proximal end and the distal end;
a fastener on the wrap for securing the free end of the flap on the compression garment for holding the wrap on the body part in said self-retaining configuration; and
a grip formed on the side edge opposite the flap for gripping the respective side edge when wrapping the wrap around the body part and fastening the fastener to hold the free end of the flap on the compression garment for holding the wrap on the body part, said grip being positioned on the wrap so that it is longitudinally offset with respect to the flap, permitting the flap to be fastened over the side edge having the grip without lapping the flap over the grip.

2. A compression garment as set forth in claim 1 wherein the grip comprises an opening in the wrap sized and shaped for receiving a finger of a user to hold the wrap on the body part.

3. A compression garment as set forth in claim 2 wherein the opening comprises a hole extending entirely through the wrap.

4. A compression garment as set forth in claim 1 wherein:
the grip comprises at least two openings in the wrap, each opening being sized and shaped for receiving a respective finger of a user to hold the wrap on the body part; and
the openings are longitudinally offset with respect to the flap and spaced from each other by a distance sufficient to receive the flap therebetween.

5. A compression garment as set forth in any one of claims 1-4 wherein at least a portion of the fastener is attached to the flap.

6. A compression garment as set forth in any one of claims 1-5 wherein the fastener is a two-part fastener, a first part of the fastener being attached to the flap and a second part of the fastener being attached to the wrap adjacent the grip, permitting the first part of the fastener to be fastened to the second part of the fastener without lapping the flap over the grip.

7. A compression garment as set forth in any one of claims 1-5 wherein:
said flap is a first flap and the garment further comprises a second flap spaced distally from said first flap by a first distance and a third flap spaced distally from said second flap by a second distance; and
said fastener is a first fastener and the garment further comprises a second fastener at least a portion of which is attached to said second flap and a third fastener at least a portion of which is attached to said third flap.

8. A compression garment as set forth in claim 7 wherein said grip is a first grip and the garment further comprises a second grip spaced distally from said first grip by said first distance and a third grip spaced distally from said second grip by said second distance.

9. A compression garment as set forth in claim 8 wherein each grip of said first, second, and third grips comprises a pair of openings in the wrap, each opening being sized and shaped for receiving a respective finger of a user to hold the wrap on the body part, each opening of each pair of openings being longitudinally offset with respect to the respective flap and spaced from each other by a distance sufficient to receive the respective flap therebetween.

10. A compression garment as set forth in claim 3 wherein:
the hole is a first hole;
the garment comprises a second hole sized and shaped for receiving a finger of a user to hold the wrap on the body part; and
said first hole and said second hole are longitudinally offset with respect to the flap and spaced from each other by a distance sufficient to receive the flap therebetween.

11. A method of placing a compression garment on a body part for providing compression therapy to the body part, the compression garment including a bladder having an inflatable chamber and a port for providing fluid communication with the inflatable chamber, the method comprising:
wrapping the compression garment around the body part such that a first side margin of the compression garment overlaps a second side margin of the compression garment;
inserting a first finger into a first opening in the second side margin to grip the second side margin;
pulling at least one of the first side margin and the second side margin while gripping the first side margin to tighten the compression garment around the body part; and
securing the tightened compression garment around the body part.

12. A method as set forth in claim 11 wherein:
the wrapping step includes lapping a flap defined by the first side margin over the second side margin; and
the pulling step includes pulling the flap across the second side margin while gripping the first side margin to tighten the compression garment around the body part.

13. A method as set forth in claims 12 or 13 further comprising:
inserting a second finger in a second opening to grip the second side margin; and
wherein the pulling step includes pulling the flap between the first and second fingers when inserted in the respective openings.
